(19) Europäisches Patentamt
European Patent Office
Office européen des brevets

(11) Veröffentlichungsnummer: **0 286 575**
**A2**

(12) # EUROPÄISCHE PATENTANMELDUNG

(21) Anmeldenummer: **88730055.6**

(22) Anmeldetag: **08.03.88**

(51) Int. Cl.⁴: **C 07 D 457/12**
C 07 D 457/04,
C 07 D 457/02, A 61 K 31/48

(30) Priorität: **10.03.87 DE 3708028**

(43) Veröffentlichungstag der Anmeldung:
**12.10.88 Patentblatt 88/41**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Heindl, Josef, Dr.**
**Eitel-Fritz-Strasse 40**
**D-1000 Berlin 38 (DE)**

**Sauer, Gerhard, Dr.**
**Königsbacher Zeile 41A**
**D-1000 Berlin 28 (DE)**

**Albrecht, Rudolf, Dr.**
**Wunsiedeler Weg 37**
**D-1000 Berlin 46 (DE)**

**Schwartz, Wolfgang**
**Daberkowstrasse 4a**
**D-1000 Berlin 20 (DE)**

**Huth, Andreas, Dr.**
**Kirchweg 55**
**D-1000 Berlin 38 (DE)**

(54) **Verfahren zur Herstellung von 2,3Beta-Dihydroergolinen, 2-substituierte 2,3Beta-Dihydroergoline und ihre Verwendung als Arzneimittel.**

(57) Es wird ein neues Verfahren zur Herstellung von 2,3β-Dihydroergolinderivaten der allgemeinen Formel I beschrieben,

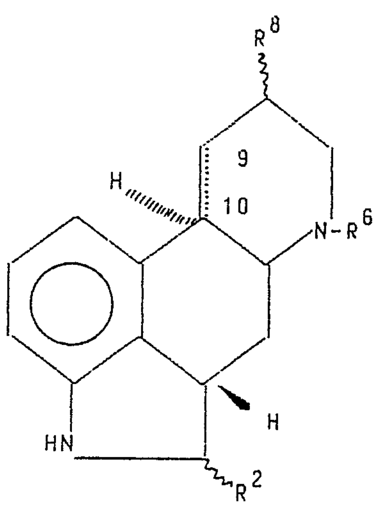

I

EP 0 286 575 A2

worin
$R^2$ Wasserstoff oder $C_{1-4}$ Alkyl,
$R^6$ $C_{1-4}$ Alkyl und
$R^8$ Methyl, Carboxymethyl, Ureido oder N, N-Diethylureido in α- oder β-Stellung und $C \underset{9}{\overset{--}{-}} C_{10}$ eine Einfach- oder Doppelbindung bedeutet, dadurch gekennzeichnet, daß man ein Ergolinderivat der allgemeinen Formel II

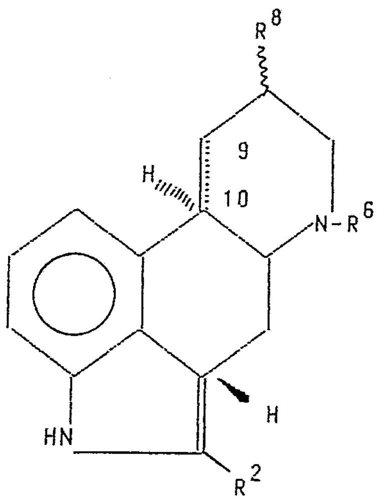

II

worin
$R^2$, $R^6$, $R^8$ und $C \underset{9}{\overset{--}{-}} C_{10}$ die oben genannte Bedeutung haben in Gegenwart einer Säure und eines $H_2$-Edelmetallkatalysators oder Organylsilans reduziert und neue 2 substituierte 2,3β-Dihydroergoline sowie deren Verwendung zu Herstellung eines Arzneimittels.

**Beschreibung**

**Verfahren zur Herstellung von 2,3β-Dihydroergolinen, 2-substituierte 2,3β-Dihydroergoline und ihre Verwendung als Arzneimittel**

Die Erfindung betrifft das Verfahren zur Herstellung von 2,3β-Dihydroergolinen, 2-substituierte 2,3β-Dihydroergolinderivate sowie deren Verwendung zur Herstellung eines Arzneimittels.

Die erfindungsgemäß herstellbaren 2,3β-Dihydroergoline sind pharmakologisch wirksame Verbindungen (DE OS 3309493) oder können als Zwischenprodukte zur Herstellung von pharmakologisch wirksamen Verbindungen verwendet werden (zum Beispiel DE OS 3533675).

Die Herstellung von in 2-Stellung unsubstituierten 2,3-Dihydroergolinen ist beispielsweise in der DEOS 2810774 und der DEOS 3411981 beschrieben. Hiernach wird das Ergolinderivat mit Natriumborhydrid in Trifluoressigsäure reduziert. Nach diesem Verfahren werden die 2,3-Dihydroergoline meist als Gemische aus 3β-H- und 3α-H-Isomeren erhalten, wobei das Isomerenverhältnis von der Struktur der Ausgangsprodukte abhängt und das Isomerengemisch als Öl anfällt. Die Hydrierung mit $NaBH_4/CF_3COOH$ ist auch durch die Explosionsneigung des Reagenzgemisches erschwert.

Aufgabe der vorliegenden Erfindung war es daher, ein Verfahren zur Herstellung von 2,3β-Dihydroergolinen zu entwickeln, das stereoselektiv in guten Ausbeuten das gewünschte Endprodukt ohne aufwendige Trennoperationen und ohne Verwendung eines gefährlichen Reagenzgemisches zugänglich macht.

Es wurde nun überraschenderweise gefunden, daß die stereoselektive Reduktion der 2,3-Doppelbindung an Ergolinderivaten in Gegenwart von Säuren mit Wasserstoff/Edelmetallkatalysatoren oder mit Organylsilanen in einfacher Weise die erfindungsgemäße Aufgabe löst.

Die Erfindung betrifft das Verfahren zur Herstellung von 2,3β-Dihydroergolinderivaten der allgemeinen Formel I

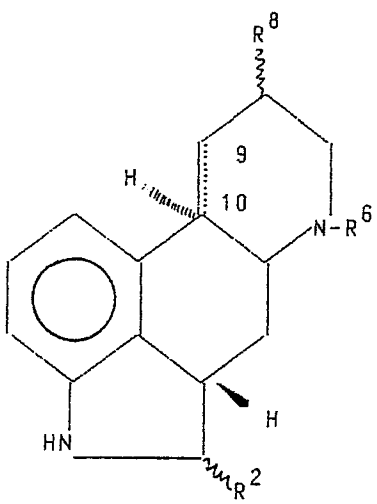

I

worin

$R^2$ Wasserstoff oder $C_{1-4}$ Alkyl,

$R^6$ $C_{1-4}$ Alkyl,

$R^8$ Methyl, Carboxymethyl, Ureido oder N, N-Diethylureido in α- oder β-Stellung und $C_9 \overset{..}{=} C_{10}$ eine C-C-Einfach- oder eine C=C-Doppelbindung bedeuten, dadurch gekennzeichnet, daß man ein Ergolinderivat der allgemeinen Formel II

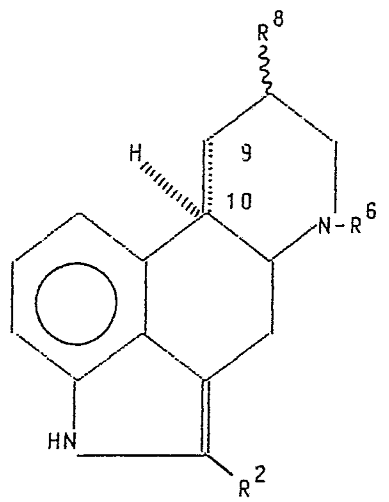

II

worin
$R^2$, $R^6$, $R^8$ und $C_9 \overset{---}{\cdot} C_{10}$ die oben genannte Bedeutung haben in Gegenwart einer Säure mit Organylsilanen oder mit Wasserstoff/Edelmetallkatalysatoren reduziert.

Als Alkylreste mit 1-4 Kohlenstoffatomen seien beispielsweise genannt Methyl, Ethyl, Propyl, Isopropyl, n-Butyl, Isobutyl, tert.-Butyl.

Als Organylsilane sind insbesondere Triarylsilane und Trialkylsilane wie beispielsweise Triphenylsilan, Trimethylsilan, Triethylsilan u.a. geeignet.

Die Reduktion mit Organylsilanen wird in Gegenwart von organischen Säuren wie beispielsweise der Trifluoressigsäure oder Essigsäure bei Temperaturen im Bereich von -70° C bis +70° C - bevorzugt zwischen -10° C bis +70° C - durchgeführt und ist im allgemeinen nach 1-3 Stunden beendet.

Die Umsetzung erfolgt in der entsprechenden Säure gegebenenfalls auch unter Zusatz eines geeigneten Lösungsmittels.

Wird die Reduktion mit Edelmetallkatalysatoren vorgenommen, so seien als Edelmetallkatalysatoren beispielsweise Platindioxid und Palladium genannt, die gegebenenfalls auf Trägern wie Kohle verwendet werden können, wobei bei Normaldruck oder erhöhtem $H_2$-Druck hydriert werden kann.

Die Reaktion wird bei Temperaturen im Bereich von -70° C bis +70° C durchgeführt und ist im allgemeinen nach 5-20 Stunden beendet.

Für die Edelmetall-katalysierte Reduktion sind als Säuren starke Mineralsäuren wie beispielsweise Tetrafluorborsäure, Schwefelsäure, Bromwasserstoffsäure, Perchlorsäure und andere geeignet. Zweckmäßigerweise arbeitet man mit 10-50 %igen Säurenkonzentrationen, wobei 20-30 %ige Säurekonzentrationen als bevorzugt anzusehen sind. Der pH-Wert des Reaktionsgemisches liegt im allgemeinen bei 0-2.

Die Umsetzung wird bevorzugt in protischen Lösungsmitteln wie beispielsweise Eisessig, Wasser und Alkoholen, zum Beispiel Methanol, Ethanol, Propanol u.a. vorgenommen.

Die nach dem erfindungsgemäßen Verfahren stereoselektiv erhaltenen 2,3β-Dihydroergolinderivate, enthalten auch in 2-Stellung kein Isomerengemisch.

Nach dem erfindungsgemäßen Verfahren können auf einfachem Wege und in sehr guten Ausbeuten sowohl die bekannten 2,3β-Dihydroergolinderivate mit $R^2$ in der Bedeutung von Wasserstoff wie auch die neuen in 2-Stellung substituierten 2,3β-Dihydroergolinderivate der allgemeinen Formel I a

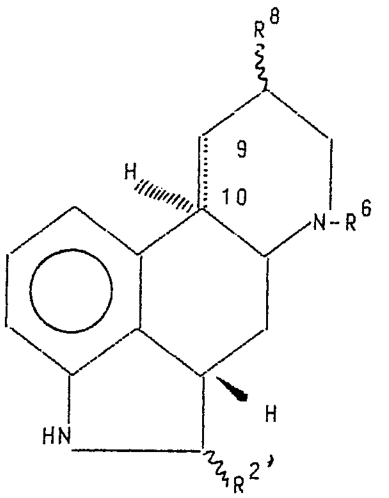

(Ia)

worin $R^6$, $R^8$ und $C_9 \overset{---}{=} C_{10}$ die oben angegebene Bedeutung haben und $R^{2'}$ $C_{1-4}$ Alkyl darstellt, erhalten werden.

Die neuen 2-substituierten 2,3β-Dihydroergolinderivate können nach den üblichen Methoden in die bekannten physiologisch verträglichen Säureadditionssalze überführt werden. Beispielsweise werden zur Bildung von Salzen die Verbindungen der allgemeinen Formel Ia in wenig Methanol oder Methylenchlorid gelöst und mit einer konzentrierten Lösung der gewünschten Säure in Methanol bei Raumtemperatur versetzt. Als Säuren geeignet sind zum Beispiel anorganische Säuren wie beispielsweise Chlorwasserstoffsäure, Salpetersäure, Phosphorsäure, Schwefelsäure, Bromwasserstoffsäure, Jodwasserstoffsäure, salpetrige Säure oder phosphorige Säure, oder organische Säuren, wie beispielsweise aliphatische Mono-oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säuren sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Fomiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzosulfonat, Toluolsulfonat, Chlorbenzolsulfonat, Xylolsulfonat, Phenylacetat, Phenylpropionat, Phenylbutyrat, Citrat, Lactat, B-Hydroxybutyrat, Glycollat, Malat, Tartrat, Methansulfonat, Propansulfonat, Naphthalin-1-sulfonat oder Naphthalin-2-sulfonat.

Die erfindungsgemäßen Verbindungen der allgemeinen Formel Ia zeigen milde dopaminerge und noradrenerge Wirkungen auf das Zentralnervensystem. Sie eignen sich daher insbesondere zur Behandlung von Altersbeschwerden.

Ferner sind die Verbindungen der Formel Ia als Zwischenprodukte zur Herstellung pharmakologisch wirksamer Verbindungen geeignet.

Zur Verwendung der erfindungsgemäßen Verbindungen als Arzneimittel werden diese in die Form eines pharmazeutischen Präparats gebracht, das neben dem Wirkstoff für die enterale oder parentale Applikation geeignete phamazeutische, organische oder anorganische inerte Trägermaterialien, wie zum Beispiel Wasser, Gelatine, Gummi, arabicum, Milchzucker Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs-, Stabilisierungs-, Netzmittel oder Emulgatoren, Salze zur Veränderung des osmotischen Drucks oder Puffer.

Die nachfolgenden Beispiele sollen das erfindungsgemäße Verfahren erläutern.

## Beispiel 1

Eine Lösung von 80 g 1,1-Diethyl-3-(6-methyl-8α-ergolinyl)harnstoff (Transdihydrolisurid) in 640 ml Ethanol wird unter Eiskühlung mit 640 ml 50 %iger Tetrafluorborsäure versetzt und die Mischung in Gegenwart von 8 g Platindioxid-Katalysator unter Schütteln 10 Stunden bei Raumtemperatur und Normaldruck hydriert. Die Reaktionsmischung wird filtriert, der Filterrückstand mit 400 ml Ethanol und 800 ml Wasser gewaschen und das Filtrat auf 8 l Eiswasser gegossen. Die Mischung wird mit konzentrierter Ammoniak-Lösung alkalisch gestellt, bis zur Sättigung mit Kochsalz versetzt und 5x mit je 1,5 l Dichlormethan extrahiert. Die vereinigten organischen Phasen werden über Natriumsulfat getrocknet und eingeengt. Es werden 79,8 g = 99 % der Theorie als Rohprodukt erhalten, das zu 95 % den 1,1-Diethyl-3-(2,3β-dihydro-6-methyl-8α(ergolinyl)-harnstoff enthält. Nach Kristallisation aus Ethylacetat werden 40,2 g = 80 % der Theorie reiner 1,1-Diethyl-3-(2,3β-dihydro-6-methyl-8α-ergolinyl)-harnstoff erhalten.

$[\alpha]_D$ = + 56° (0,5 % in Chloroform)

Beispiel 2

Analog Beispiel 1 wird hergestellt:
aus 6-Methyl-8β-ergolincarbonsäuremethylester der 2,3β-Dihydro-6-methyl-8β-ergolincarbonsäuremethylester in 82 % Ausbeute,
aus 1,1-Diethyl-3-(6-n-propyl-8α-ergolinyl)-harnstoff der 1,1-Diethyl-3-(2,3β-dihydro-6-n-propyl-8α-ergolinyl)-harnstoff in 75 % Ausbeute,
aus 1,1-Diethyl-3-(6-methyl-8β-ergolinyl)-harnstoff der 1,1-Diethyl-3-(2,3β-dihydro-6-methyl-8β-ergolinyl)-harnstoff in 73 % Ausbeute,
aus Festuclavin (6,8β-Dimethyl-ergolin) das 2,3β-Dihydro-6,8-dimethyl-ergolin in 84 % Ausbeute,

Beispiel 3

Eine Lösung von 200 mg 1,1-Diethyl-3-(6-methyl-8α-ergolinyl)-harnstoff (Transdihydrolisurid) in 2 ml Trifluoressigsäure wird auf 60° C erhitzt, mit 0,18 ml Triethylsilan versetzt und die Mischung 2 Stunden bei 60° C gerührt. Die Reaktionsmischung wird auf Eis gegeben, mit konz. Ammoniaklösung alkalisiert und mit Dichlormethan ausgeschüttelt. Die organischen Phasen werden getrocknet, eingeengt und der Rückstand am Ethylacetat/Pentan kristallisiert. Man erhält so 185 mg = 92 % der Theorie 1,1-Diethyl-3-(2,3β-dihydro-6-methyl-8α-ergolinyl)-harnstoff.
$[8\alpha]_D$ = +56° (0,5 % in Chloroform)

Beispiel 4

Analog Beispiel 3 wird dargestellt:
aus 1,1-Diethyl-3-(2,6-dimethyl-8α-ergolinyl)-harnstoff der 1,1-Diethyl-3-(2,3β-dihydro-2,6-dimethyl-8α-ergolinyl)-harnstoff in 92 % Ausbeute.
$[\alpha]_D$ = +54° (0,5 in Chloroform).
aus 1,1-Diethyl-3-(2-ethyl-6-methyl-8α-ergolinyl)-harnstoff der 1,1-Diethyl-3-(2-ethyl-2,3-β-dihydro-6-methyl-8α-ergolinyl)-harnstoff in 86 % Ausbeute.
$[\alpha]_D$ = +70° ( 0,175 % in Pyridin).
aus 3-(9,10-Didehydro-6-methyl-8α-ergolinyl)-1,1-diethyl-harnstoff der 3-(9,10-Didehydro-2,3β-dihydro-6-methyl-8α-ergolinyl)-1,1-diethylharnstoff in 70 % Ausbeute.
$[\alpha]_D$ = +208° (0,5 % in Chloroform)

**Patentansprüche**

1.) Verfahren zur Herstellung von 2,3β-Dihydroergolinderivaten der allgemeinen Formel I

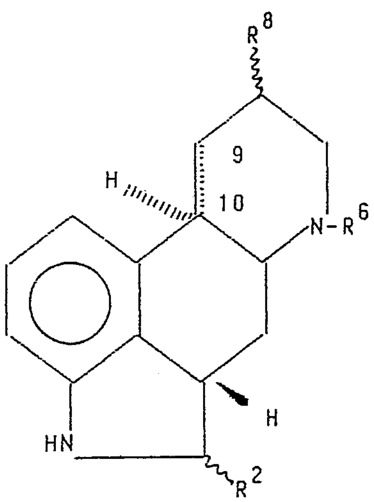

I

worin
$R^2$ Wasserstoff oder $C_{1-4}$ Alkyl,
$R^6$ $C_{1-4}$ Alkyl und
$R^8$ Methyl, Carboxymethyl, Ureido oder N, N-Diethylureido in α- oder B-Stellung und $C_9 \overset{---}{} C_{10}$ eine Einfach- oder Doppelbindung bedeutet, dadurch gekennzeichnet, daß man ein Ergolinderivat der

allgemeinen Formel II

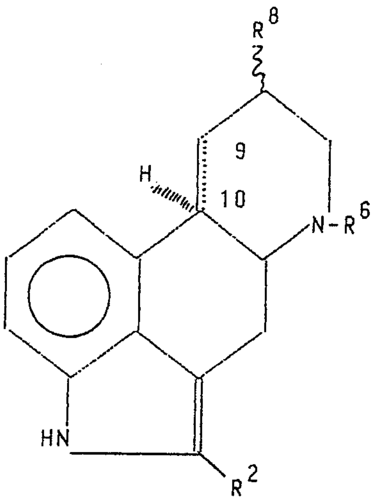

II

worin $R^2$, $R^6$, $R^8$ und $C_9 \overset{---}{=} C_{10}$ die oben genannte Bedeutung haben in Gegenwart einer Säure und eines Organylsilans oder $H_2$/Edelmetallkatalysators reduziert.

2.) 2-substituierte 2,3β-Dihydroergolinderivate der allgemeinen Formel Ia

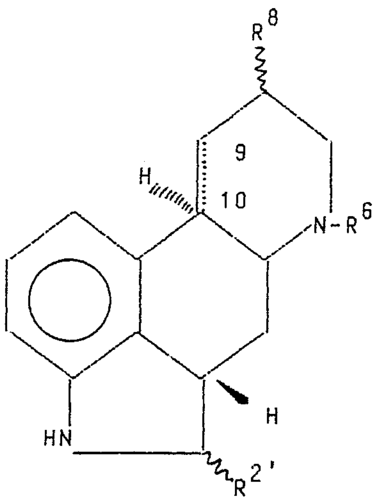

(Ia)

worin $R^6$, $R^8$ und $C_9 \overset{---}{=} C_{10}$ die in Anspruch 1 genannte Bedeutung haben und $R^{2'}$ $C_{1-4}$ Alkyl ist und deren Säureadditionssalze.

3.) 1,1-Diethyl-3-(2,3β-dihydro-2,6-dimethyl-8α-ergolinyl)-harnstoff
1,1-Diethyl-3-(2-ethyl-2,3β-dihydro-6-methyl-8α-ergolinyl)-harnstoff
3-(9,10-Didehydro-2,3β-dihydro-6-methyl-8α-ergolinyl)-1,1-diethylharnstoff

4.) Arzneimittel auf Basis der Verbindungen gemäß Anspruch 2 und 3.